# EUROPÄISCHE PATENTSCHRIFT

(11) **EP 1 549 603 B1**
(45) Veröffentlichungstag und Bekanntmachung des Hinweises auf die Patenterteilung: **10.02.2010**
(21) Anmeldenummer: 03764917.5
(22) Anmeldetag: 07.06.2003
(51) Int. Cl.: C07C 51/36, B01J 35/10, B01J 23/46, B01J 21/06

(54) **FEINPORIGER KATALYSATOR UND VERFAHREN ZUR HYDRIERUNG VON AROMATISCHEN VERBINDUNGEN**
MICROPOROUS CATALYST AND METHOD FOR HYDROGENATING AROMATIC COMPOUNDS
CATALYSEUR A MICROPORES ET PROCEDE D'HYDROGENATION DE COMPOSES AROMATIQUES

(30) Priorität: 19.07.2002 DE 10232868
(43) Veröffentlichungstag der Anmeldung: 06.07.2005
(73) Patentinhaber: Evonik Oxeno GmbH, 45772 Marl (DE)
(72) Erfinder: GRASS, Michael, 45721 Haltern am See (DE); KAIZIK, Alfred, 45772 Marl (DE); BÜSCHKEN, Wilfried, 45721 Haltern am See (DE); TUCHLENSKI, Axel, 69469 Weinheim (DE); MASCHMEYER, Dietrich, 45657 Recklinghausen (DE); GAUDSCHUN, Kurt-Alfred, 45665 Recklinghausen (DE); BROCKSIEN, Frank, 48249 Dülmen (DE)
(74) Vertreter: Hirsch, Hans-Ludwig
(86) Internationale Anmeldenummer: PCT/EP2003/006002
(87) Internationale Veröffentlichungsnummer: WO 2004/009526

(56) Entgegenhaltungen:
- WO-A-02/43862
- WO-A-99/32427
- DE-A- 4 419 974
- US-A- 4 422 960
- US-A- 5 558 766

## Beschreibung

Die Erfindung betrifft die Hydrierung von aromatischen Verbindungen, insbesondere diewherein the activation system is designed such that before the container (2; 9) hits the at least one securing wall (1) the at least one airbag (3) can be activated, Herstellung von alicyclischen Polycarbonsäuren oder deren Ester durch Kernhydrierung der entsprechenden aromatischen Polycarbonsäuren oder deren Ester, sowie hierfür geeignete feinporige Katalysatoren.

Alicyclische Polycarbonsäureester, wie beispielsweise die Ester der Cyclohexan-1,2-dicarbonsäure, werden als Schmierölkomponente und als Hilfsmittel bei der Metallverarbeitung eingesetzt. Weiterhin finden sie als Weichmacher für Polyolefine und für PVC Verwendung.

Für die Weichmachung von PVC werden überwiegend Ester der Phthalsäure, wie beispielweise Dibutyl- , Dioctyl-, Dinonyl- oder Didecylester, verwendet. Da über die Verwendung dieser Phthalate in letzter Zeit zunehmend kontrovers diskutiert wird, könnte deren Einsatz in Kunststoffen eingeschränkt werden. Alicyclische Polycarbonsäureester, von denen einige in der Literatur bereits als Weichmacher für Kunststoffe beschrieben sind, könnten dann als geeignete Ersatzstoffe zur Verfügung stehen.

In den meisten Fällen ist der wirtschaftlichste Weg zur Herstellung von alicyclischen Polycarbonsäureestern die Kernhydrierung der entsprechenden aromatischen Polycarbonsäureester, beispielsweise der o. g. Phthalate. Es sind hierzu bereits einige Verfahren bekannt:

In US 5 286 898 und US 5 319 129 werden Verfahren beschrieben, mit denen Dimethylterephthalat an geträgerten Pd-Katalysatoren, die mit Ni, Pt und/oder Ru dotiert sind, bei Temperaturen größer oder gleich 140 °C und einem Druck zwischen 50 und 170 bar zum entsprechenden Hexahydrodimethylterephthalat hydriert werden kann.

US 3 027 398 offenbart die Hydrierung von Dimethylterephthalat an geträgerten Ru-Katalysatoren bei 110 bis 140 °C und 35 bis 105 bar.

In DE 28 23 165 werden aromatische Carbonsäureester an geträgerten Ni, Ru, Rh und/oder Pd-Katalysatoren zu den entsprechenden alicyclischen Carbonsäureestern bei 70 bis 250°C und 30 bis 200 bar hydriert. Dabei wird ein makroporöser Träger mit einer mittleren Porengröße von 70 nm und einer BET-Oberfläche von ca. 30 m²/g eingesetzt.

In WO 99/32427 und WO 00/78704 werden Verfahren zur Hydrierung von Benzolpolycarbonsäureestem zu den entsprechenden alicyclischen Verbindungen offengelegt. Dabei werden Trägerkatalysatoren eingesetzt, die ein Metall der VIII. Nebengruppe alleine oder zusammen mit mindestens einem Metall der I. oder VII. Nebengruppe des Periodensystems der Elemente enthalten und Makroporen aufweisen. Als bevorzugtes Metall der VIII. Nebengruppe wird Ruthenium eingesetzt. Zur Hydrierung werden drei verschiedene Katalysatortypen eingesetzt, die sich im Wesentlichen durch ihre mittleren Porendurchmesser und ihre BET-Oberflächen unterscheiden.
- Katalysator I:: mittlerer Porendurchmesser größer 50 nm und BET-Oberfläche kleiner 30 m²/g
- Katalysator II:: mittlerer Porendurchmesser 5 bis 20 nm und BET-Oberfläche größer 50 m²/g.
- Katalysator III:: mittlerer Porendurchmesser größer 100 nm und BET-Oberfläche kleiner 15 m²/g

Neben dem Porendurchmesser wird das durch Poren eines bestimmten Durchmessers gebildete Porenvolumen angegeben. Die bei der Herstellung von Katalysator II eingesetzten Trägermaterialien weisen eine Porenverteilung auf, bei der ca. 5 bis ca. 50 % des Porenvolumens von Makroporen (Durchmesser ca. 50 nm bis 10.000 nm) und ca. 70 - ca. 90 % des Porenvolumens von Mesoporen (Durchmesser ca. 2 bis 50 nm) auf. Der mittlere Porendurchmesser liegt zwischen ca. 5 und 20 nm.

In den Dokumenten US-A-5588766, WO 02/43862 A und US-A-4422960 werden Katalysatoren für die Wasserstoffbehandlung von Erdölprodukten beschrieben, welche poröse Trägermaterialien auf Basis von Metalloxiden wie insbesondere Aluminumoxid umfassen. Die Materialien der letztgenannten Schrift können unter anderem auch Titandioxid enthalten.

Die Aktivität und Selektivität von Hydrierkatalysatoren hängt von deren Oberflächeneigenschaften wie Porengröße, BET-Oberfläche oder Oberflächenkonzentration der aktiven Metalle ab.

Die für die Kernhydrierung von aromatischen Carbonsäuren oder deren Ester eingesetzten Katalysatoren sollten eine hohe Reaktionsgeschwindigkeit ermöglichen, nur einen geringen Anteil an Nebenprodukten generieren und eine lange Standzeit aufweisen.

Nun ist ein Katalysator in einem kontinuierlich betriebenen Verfahren mechanischen, thermischen und chemischen Belastungen ausgesetzt, die die Porengröße bzw. die BET-Oberfläche verändern und damit die Aktivität und Selektivität dieses Katalysators herabsetzen.

So ist an vielen Katalysatoren neben der mechanischen Abrasion ein Vergrößern der Porenvolumina und Durchmesser durch Säurefraß zu beobachten.

Aromatische Polycarbonsäureester enthalten häufig geringe Mengen an Carbonsäuren, zusätzlich entstehen während der Kernhydrierung von Estern Säurespuren. Partialester von Polycarbonsäuren oder Polycarbonsäuren als solche sind aufgrund ihrer Struktur acide. Daher sollte ein für ein kontinuierliches Verfahren geeigneter Hydrierkatalysator auch bei höheren Temperaturen unter den Hydrierbedingungen gegen Säure beständig sein.

Weiterhin sind die Oberflächeneigenschaften der Katalysatoren für deren Reaktivität verantwortlich. Die bekannten Katalysatoren sind hier noch verbesserungsfähig.

Es wurde nun überraschend gefunden, dass Katalysatoren, die mindestens ein Metall der achten Nebengruppe des Periodensystems der Elemente enthalten und aus einem Trägermaterial aus Titandioxid, welches zusätzlich Alkalimetalle, Erdalkalimetalle und/oder Schwefel enthalten kann, mit einem mittleren Porendurchmesser von 2 bis 50 nm und einer engen Porenverteilung mit feinporiger Oberflächenstruktur bestehen, aromatische Carbonsäuren und/oder deren Ester (Voll- oder Partialester) in hoher Selektivität und Raum-Zeit-Ausbeute ohne nennenswerte Nebenreaktionen zu den entsprechenden alicyclischen Polycarbonsäuren oder deren Estern hydrieren.

Gegenstand der vorliegenden Erfindung ist daher ein Katalysator zur Hydrierung aromatischer Poly- und/oder Monocarbonsäuren oder deren Derivate zu den entsprechenden alicyclischen Verbindungen, der mindestens ein Metall der achten Nebengruppe des Periodensystems der Elemente auf oder in einem Trägermaterial enthält, wobei das Trägermaterial aus Titandioxid besteht, welches zusätzlich Alkalimetalle, Erdalkalimetalle und/oder Schwefel enthalten kann, und einen mittleren Porendurchmesser von 2 bis 50 nm aufweist und über 95 % des Gesamtporenvolumens der Trägermaterialien auf Poren mit einem Durchmesser von kleiner 50 nm entfällt.

Katalysatoren dieser Art können besonders zur Hydrierung von aromatischen Verbindungen verwendet werden. Ein Verfahren zur katalytischen Hydrierung von aromatischen Poly- und/oder Monocarbonsäuren oder deren Derivate mit Wasserstoff-haltigen Gasen an einem Katalysator, der mindestens ein Metall der achten Nebengruppe des Periodensystems auf oder in einem Trägermaterial enthält, wobei das Trägermaterial aus Titandioxid besteht, welches zusätzlich Alkalimetalle, Erdalkalimetalle und/oder Schwefel enthalten kann, und einen mittleren Porendurchmesser von 2 bis 50 nm aufweist und über 95 % des Gesamtporenvolumens der Trägermaterialien auf Poren mit einem Durchmesser von kleiner 50 nm entfällt, ist ebenfalls Gegenstand der vorliegenden Erfindung.

Die Katalysatoren können prinzipiell jedes Metall der achten Nebengruppe des Periodensystems der Elemente enthalten. Vorzugsweise werden als Aktivmetalle Platin, Rhodium, Palladium, Kobalt, Nickel oder Ruthenium oder ein Gemisch aus zwei oder mehreren davon eingesetzt, wobei insbesondere Ruthenium als Aktivmetall eingesetzt wird.

Neben den bereits genannten Metallen kann zusätzlich mindestens ein Metall der ersten und/oder siebten Nebengruppe des Periodensystems der Elemente in den Katalysatoren enthalten sein. Bevorzugt wird Rhenium und/oder Kupfer eingesetzt.

Der Gehalt der Aktivmetalle, d. h. der Metalle der ersten und/oder siebten und/oder achten Nebengruppe des Periodensystems der Elemente beträgt im Allgemeinen 0,1 bis 30 Massen-%. Der Edelmetallgehalt, d. h. der Metalle der achten Nebengruppe des Periodensystems der Elemente und der fünften oder sechsten Periode, z. B. Palladium, Ruthenium, berechnet als Metall liegt im Bereich von 0,1 bis 10 Massen-%, insbesondere im Bereich von 0,8 bis 5 Massen-%, ganz besonders zwischen 1 und 3 Massen-%.

Zur Herstellung der erfindungsgemäßen Katalysatoren werden Trägermaterialien mit einem mittleren Porendurchmesser, der im Bereich von 2 bis 50 nm liegt, verwendet. (Die Bestimmung des mittleren Porendurchmesser erfolgt durch Hg-Porosimetrie, insbesondere nach DIN 66133.)

Bei den eingesetzten Trägermaterialien kann zwischen Mikroporen (Porendurchmesser kleiner 2 nm), Mesoporen (Porendurchmesser 2 bis 50 nm) und Makroporen (Porendurchmesser größer 50 nm) unterschieden werden. So sind hinsichtlich der Porenart Trägermaterialien mit folgenden Porenkombinationen einsetzbar:
a) nur Mesoporen
b) Mikroporen und Mesoporen
c) Mesoporen und Makroporen
d) Mikroporen und Mesoporen und Makroporen
e) Mikroporen und Makroporen

Entscheidend für die Herstellung der erfindungsgemäßen Katalysatoren ist, dass unabhängig von der Porengrößenverteilung der mittlere Porendurchmesser des Trägermaterials zwischen 2 und 50 nm liegt. Vorzugsweise beträgt der mittlere Porendurchmesser 5 bis 24 nm, ganz besonders bevorzugt 10 bis 19 nm.

Die spezifische Oberfläche des Trägers ( bestimmt nach dem BET-Verfahren durch Stickstoff-Adsorption, gemäß DIN 66 131 beträgt 1-350 m²/g, bevorzugt 1-200 m²/g, besonders bevorzugt 1-100 m²/g, insbesondere 10-90 m²/g bzw. 50-80 m²/g bzw. 1-40 m²/g.

Zur Herstellung der Katalysatoren werden Trägermaterialien verwendet, bei denen über 95 %, insbesondere über 97 % des Gesamtporenvolumens auf Mikro- und Mesoporen, d. h. Poren mit einem Durchmesser von 2 bis 50 nm, entfällt.

Das Gesamtporenvolumen des erfindungsgemäßen Katalysators beträgt 0,25 bis 0,50 ml/g, insbesondere 0,28 bis 0,43 ml/g.

Als Träger für die Herstellung der erfindungsgemäßen Katalysatoren werden Feststoffe, die aus Titanoxid bestehen, eingesetzt, deren mittlere Porendurchmesser und deren spezifische Oberfläche in den oben genannten Bereichen liegen. Zusätzlich können diese Trägermaterialien Alkalimetalle, Erdalkalimetalle und/oder Schwefel enthalten.

Als Ausgangsmaterial für die Herstellung der erfindungsgemäßen Katalysatoren wird vorzugsweise ein Titanhydroxid (Metatitansäure) verwendet. Metatitansäure wird als Zwischenprodukt bei der TiO₂-Herstellung nach dem klassischen Sulfatverfahren durch Aufschluß von Ilmenit mit Schwefelsäure gewonnen (s. Ullmann's Encyklopädie der technischen Chemie, 4. Auflage, Bd. 18 (1979), S. 574 ff).
Die Schwefelsäure-haltige Metatitansäure wird nach weitgehender Entfernung der Schwefelsäure und Waschen mit entsalztem Wasser sowie partieller Peptisierung mit Salpetersäure durch Calcinieren bei 490 bis 530 °C in ein Titandioxid vom Anatas-Typ überführt.

Die Entfernung der Schwefelsäure kann durch Neutralisation mit Ammoniak oder Alkalilaugen und anschließender Wasserwäsche erfolgen. Eine andere Möglichkeit der Entsäuerung besteht darin, dass die Schwefelsäure-haltige Metatitansäure mit wasserlöslichen Bariumsalzen, wie beispielsweise Bariumnitrat, Bariumchlorid, Bariumcarbonat, umgesetzt und mit Wasser gewaschen wird. In diesem Falle wird ein Titandioxid erhalten, das Bariumsalze und gegebenenfalls geringe Mengen an Schwefelsäure bzw. Sulfat enthält.

Das so erhaltene Material wird zu den gewünschten Teilchengrößen aufgemahlen und abgesiebt. Das calcinierte TiO₂-Pulver und/oder TiO₂-Pulvergemische werden unter Zusatz von Wasser und Plastifizierhilfsmittel in einer Mischvorrichtung, wie z. B. in einem Kneter oder Rührer, homogenisiert und in einem Formgebungsapparat, wie z. B. in einem Extruder oder einer Tablettiermaschine, zu Formkörpern gewünschter Gestalt, wie Strängen oder Tabletten, geformt. Durch anschließendes Trocknen bei 80-120 °C und Calcinieren bei 450-550 °C wird der fertige TiO₂-Träger mit erfindungsgemäßer Porenstruktur hergestellt. Für die Verbesserung der mechanischen Festigkeit der TiO₂-Träger ist es vorteilhaft, wie in DE 44 19 974 beschrieben, für die Träger-Herstellung ein TiO₂-Pulvergemisch aus mindestens zwei Pulvern unterschiedlicher Teilchengrößenverteilung zu verwenden.
Bei den bevorzugt verwendeten Titandioxid-Trägern, die mit Bariumsalzen modifiziert wurden, liegt der Bariumgehalt zwischen 1,0 und 4,0 Massen-%. Der Gehalt an "freiem" Sulfat kann 1,0 - 5,5 Massen-% betragen. Unter freiem Sulfat wird Schwefel, berechnet als Sulfat der Oxidationsstufe 6 verstanden, der nicht als Bariumsulfat vorliegt. Freies Sulfat kann z. B. nach oxidativer Behandlung des Katalysatormaterials in wässriger Lösung durch Titration bestimmt werden, da hier Bariumsulfat als sehr schwerlösliches Salz nicht mitbestimmt wird.
Die erfindungsgemäßen Katalysatoren können durch Auftragen mindestens eines Metalls der achten Nebengruppe des Periodensystems der Elemente und gegebenenfalls mindestens eines Metalls der ersten und/oder siebten Nebengruppe des Periodensystems der Elemente auf einem geeigneten Träger erhalten werden. Es ist auch möglich, die Aktivmetalle und den Träger gleichzeitig herzustellen, d. h. einen Vollkatalysator einzusetzen.

Die Auftragung kann durch Tränken des Trägers in wässrigen Metallsalzlösungen, wie z. B. wässrigen Rutheniumsalzlösungen, durch Aufsprühen entsprechender Metallsalzlösungen auf den Träger oder durch andere geeignete Verfahren erreicht werden. Als Metallsalze der ersten, siebten oder achten Nebengruppe des Periodensystems eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die neben dem Metall der achten Nebengruppe des Periodensystems der Elemente noch weitere Metalle als Aktivmetall aufgetragen enthalten, können die Metallsalze bzw. Metallsalzlösungen gleichzeitig oder nacheinander aufgebracht werden.

Die mit Metallsalzlösung beschichteten bzw. getränkten Träger werden anschließend, vorzugsweise bei Temperaturen von 80 bis 150 °C, getrocknet und wahlweise bei Temperaturen von 200 bis 600 °C calciniert. Bei getrennter Auftränkung wird der Katalysator nach jedem Tränkschritt getrocknet und wahlweise calciniert, wie oben beschrieben. Die Reihenfolge, in der Aktivkomponenten aufgebracht werden, ist dabei frei wählbar.

Optional kann die Aufbringung der Aktivkomponenten, Trocknung und Calcinierung in einem Arbeitsgang erfolgen, beispielsweise durch Aufsprühen einer wässrigen Metallsalzlösung auf den Träger bei Temperaturen über 200 °C.

Die erfindungsgemäßen Katalysatoren werden zweckmäßig in eine Form gebracht, die bei der Hydrierung einen geringen Strömungswiderstand bietet, wie beispielsweise Tabletten, Zylinder, Strangextrudate oder Ringe. Die Formgebung kann dabei wahlweise an verschiedenen Stellen der Katalysatorherstellung erfolgen.

Im erfindungsgemäßen Verfahren wird die Hydrierung in flüssiger Phase oder in der Gasphase durchgeführt. Die Hydrierung kann an suspendierten oder stückigen im Festbett angeordneten Katalysatoren kontinuierlich oder diskontinuierlich durchgeführt werden. Im erfindungsgemäßen Verfahren wird eine kontinuierliche Hydrierung an einem im Festbett angeordnetem Katalysator, bei dem sich unter Reaktionsbedingungen die Produkt/Eduktphase hauptsächlich im flüssigen Zustand befindet, bevorzugt.

Wenn die Hydrierung kontinuierlich an einem im Festbett angeordneten Katalysator durchgeführt wird, ist es zweckmäßig, den Katalysator vor der Hydrierung in die aktive Form zu überführen. Dies kann durch Reduktion des Katalysators mit Wasserstoff-haltigen Gasen nach einem Temperaturprogramm erfolgen. Dabei kann die Reduktion gegebenenfalls in Gegenwart einer flüssigen Phase, die über den Katalysator rieselt, durchgeführt werden. Als flüssige Phase kann dabei ein Lösemittel oder das Hydrierprodukt eingesetzt werden.

Für das erfindungsgemäße Verfahren können unterschiedliche Verfahrensvarianten gewählt werden. Es kann adiabatisch, polytrop oder praktisch isotherm, d. h. mit einem Temperaturanstieg von typischerweise kleiner als 10 °C, ein- oder mehrstufig durchgeführt werden. Im letzteren Falle kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die aromatischen Verbindungen im geraden Durchgang oder unter Produktrückfführung zu hydrieren.

Das erfindungsgemäße Verfahren wird bevorzugt in der Flüssig/Gas-Mischphase oder Flüssigphase in Dreiphasenreaktoren im Gleichstrom durchgeführt, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und einer hohen Raum-Zeit-Ausbeute werden die Reaktoren vorzugsweise mit hohen Flüssigkeitsbelastungen von 15 bis 120, insbesondere von 25 bis 80 m³ pro m² Querschnitt des leeren Reaktors und Stunde betrieben. Wird ein Reaktor im geraden Durchgang betrieben, so kann die spezifische Katalysatorbelastung (LHSV) Werte zwischen 0,1 und 10 h⁻¹ annehmen.

Die Hydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

### Beispielsweise können folgende Stoffe als Lösemittel eingesetzt werden:

Geradkettige oder cyclische Ether, wie beispielsweise Tetrahydrofuran oder Dioxan, sowie aliphatische Alkohole, in denen der Alkylrest 1 bis 13 Kohlenstoffatome aufweist.

Bevorzugt verwendbare Alkohole sind Isopropanol, n-Butanol, Isobutanol, n-Pentanol, 2-Ethylhexanol, Nonanole, technische Nonanolgemische, Decanol, technische Decanolgemische, Tridecanole.

Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch würde die Nebenproduktbildung durch Umesterung ausgeschlossen. Ein weiteres bevorzugtes Lösemittel ist das Hydrierprodukt selbst.

Durch die Verwendung eines Lösemittels kann die Aromatenkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zur Folge haben. Bevorzugt liegt der Aromatengehalt im Reaktorzulauf zwischen 1 und 35 %, insbesondere zwischen 5 und 25 %. Der gewünschte Konzentrationsbereich kann bei Reaktoren, die in Schlaufenfahrweise betrieben werden, durch das Kreislaufverhältnis (Mengenverhältnis von rückgeführten Hydrieraustrag zu Edukt) eingestellt werden.

Das erfindungsgemäße Verfahren wird in einem Druckbereich von 3 bis 300 bar, insbesondere zwischen 15 und 200 bar, ganz besonders zwischen 50 und 200 bar durchgeführt. Die Hydriertemperaturen liegen zwischen 50 und 250, insbesondere zwischen 100 und 200 °C.

Als Hydriergase können beliebige Wasserstoff-haltige Gasgemische, die keine schädlichen Mengen an Katalysatorgiften wie beispielsweise Kohlenmonoxid oder Schwefelwasserstoff enthalten, eingesetzt werden. Die Verwendung von Inertgasen ist optional, bevorzugt wird Wasserstoff in einer Reinheit von größer 95 %, insbesondere größer 98 % eingesetzt. Inertgasbestandteile können beispielsweise Stickstoff oder Methan sein.

Die einzelnen Reaktoren können mit Frisch-Wasserstoff beschickt werden. Um den Wasserstoffverbrauch und die mit dem Abgas bedingten Austragsverluste zu minimieren, ist es jedoch zweckmäßig, das Abgas eines Reaktors als Hydriergas eines anderen Reaktors zu verwenden. Beispielsweise ist es bei einem Verfahren, das in zwei hintereinander geschalteten Reaktoren durchgeführt wird, vorteilhaft, Frisch-Wasserstoff in den zweiten Reaktor einzuspeisen und das Abgas des zweiten Reaktors in den ersten Reaktor zu leiten. In diesem Falle strömen Einsatzstoff und Hydriergas in entgegengesetzter Reihenfolge durch die Reaktoren. Es ist zweckmäßig, den Wasserstoffüberschuss bezogen auf die stöchiometrisch notwendige Menge unter 30 %, insbesondere unter 10 %, ganz besonders unter 5 % zu halten.

Werden Octyl- oder Nonylphthalate oder deren Gemische zu den entsprechenden 1.2-Cyclohexandicarbonsäureestern umgesetzt, so wird die Hydrierung vorzugsweise in der Flüssig/Gas-Mischphase oder Flüssigphase in zwei hintereinandergeschalteten Reaktoren durchgeführt. Dabei wird der erste Reaktor in Schlaufenfahrweise betrieben, d. h., dass ein Teil des Hydrieraustrags des ersten Reaktors zusammen mit Frisch-Edukt auf den Kopf des ersten Reaktors geleitet wird. Der andere Teil des Austrags des ersten Reaktors wird in einem zweiten Reaktor im geraden Durchgang hydriert. Es ist auch möglich, anstelle eines großen Schlaufenreaktors mehrere kleinere Schlaufenreaktoren, die in Reihe oder parallel angeordnet sind, zu verwenden. Ebenso ist es möglich, anstatt eines großen Reaktors, der im geraden Durchgang durchströmt wird, mehrere Reaktoren, die in Reihe oder parallel miteinander verschaltet sind, zu betreiben. Bevorzugt wird jedoch nur ein Schlaufenreaktor und nur ein Reaktor, der im geraden Durchgang betrieben wird, verwendet. Im erfindungsgemäßen Verfahren wird die Hydrierung von Octyl-, Nonyl-, Decyl- oder Dodecylphthalaten vorzugsweise unter folgenden Bedingungen durchgeführt:

Die Konzentration dieser Phthalate am Eintritt des ersten Reaktors (Schlaufenreaktor) liegt zwischen 1 und 30 Massen-%, bevorzugt zwischen 2 und 10 Massen-%, ganz besonders bevorzugt zwischen 3 und 8 Massen-%.

Im Hydrieraustrag des ersten Reaktors liegt die Konzentration der Phthalate zwischen 0,5 und 20 Massen-%, insbesondere zwischen 1 und 10 Massen-%.

Die spezifische Katalysatorbelastung (LHSV, Liter Frisch-Edukt je Liter Katalysator je Stunde) im Schlaufenreaktor beträgt 0,1 bis 5, h⁻¹ insbesondere 0,5 bis 3 h⁻¹.

Die Oberflächenbelastung im Schlaufenreaktor liegt im Bereich von 10 bis 100 m³/m²/h, bevorzugt im Bereich von 20 bis 80 m³/m²/h, ganz besonders bevorzugt im Bereich von 40 bis 60 m³/m²/h.

Die durchschnittlichen Hydriertemperaturen im Schlaufenreaktor sind 60 bis 150 °C, insbesondere 70 bis 120 °C.

Der Hydrierdruck im Schlaufenreaktor beträgt 25 bis 200, insbesondere 80 bis 110 bar

Im Austrag des zweiten Reaktors ist die Konzentration an Edukt kleiner als 0,3 Massen-%, insbesondere kleiner als 0,1 Massen-%, ganz besonders kleiner als 0,05 Massen-%.

Die spezifische Katalysatorbelastung im zweiten Reaktor ( Liter Nonylphthalat je Liter Katalysator je Stunde) beträgt 1 bis 20 h⁻¹, insbesondere 2 bis 10 h⁻¹.

Im zweiten Reaktor liegt die durchschnittliche Temperatur zwischen 60 und 150 °C, insbesondere 70 und 120 °C.

Der Hydrierdruck im zweiten Reaktor beträgt 25 bis 200 bar, insbesondere 80 bis 110 bar. Nach dem erfindungsgemäßen Verfahren werden aromatische Poly- und/oder Monocarbonsäuren oder deren Derivate, insbesondere deren Alkylester zu den entsprechenden alicyclischen Polycarbonsäureverbindungen umgesetzt. Dabei können sowohl Vollester als auch Partialester hydriert werden. Unter Vollester wird eine Verbindung verstanden, bei der alle Säuregruppen verestert sind. Partialester sind Verbindungen mit mindestens einer freien Säuregruppe (oder ggf. einer Anhydridgruppe) und mindestens einer Estergruppe.

Werden im erfindungsgemäßen Verfahren Polycarbonsäureester eingesetzt, so enthalten diese bevorzugt 2, 3 oder 4 Esterfunktionen.

Die im erfindungsgemäßen Verfahren verwendeten aromatischen Poly- und/oder Monocarbonsäuren bzw. Polycarbonsäureester sind bevorzugt Benzol-, Diphenyl-, Naphthalin- und/oder Anthracenpolycarbonsäuren, deren Anhydride und/oder die entsprechenden Ester. Die so erhaltenen alicyclischen Polycarbonsäuren bzw. deren Derivate bestehen aus einem oder mehreren, ggf. durch eine C-C-Bindung verknüpfte oder ankondensierte C₆-Ringen.

Die Alkoholkomponente der eingesetzten Carbonsäureester besteht bevorzugt aus verzweigten oder unverzweigten Allyl-, Cycloalkyl- oder Alkoxyalkylgruppen mit 1 - 25 Kohlenstoffatomen. Diese können in einem Molekül eines Polycarbonsäureesters gleich oder unterschiedlich sein, d. h. sie können gleiche oder verschiedene Isomeren oder Kettenlängen besitzen. Selbstverständlich können auch Isomere bezüglich des Substitutionsmusters des aromatischen Systems in Form eines Gemisches eingesetzt werden, z. B. ein Gemisch aus Phthalsäureester und Terephthalsäureester.

In einer bevorzugten Ausführungsform betrifft die vorliegende Erfindung ein Verfahren zur Hydrierung der 1,2-; 1,3- oder 1,4-Benzoldicarbonsäureester, und/oder der 1,2,3-; 1,2,4- oder 1,3,5-Benzoltricarbonsäureester, d. h. es werden die Isomere der 1,2-; 1,3- oder 1,4-Cyclohexandicarbonsäureester, oder der 1,2,3-; 1,3,5- oder 1,2,4-Cyclohexantricarbonsäureester erhalten.

Im erfindungsgemäßen Verfahren können beispielsweise Ester folgender aromatischer Carbonsäuren eingesetzt werden: 1,2-Naphthalindicarbonsäure, 1,3-Naphthalindicarbonsäure, 1,4-Naphthalindicarbonsäure, 1,5-Naphthalindicarbonsäure, 1,6-Naphthalindicarbonsäure, 1,7-Naphthalindicarbonsäure, 1,8-Naphthalindicarbonsäure, Phthalsäure (Benzol-1,2-dicarbonsäure), Isophthalsäure (Benzol-1,3-dicarbonsäure), Terephthalsäure (Benzol-1,4-dicarbonsäure), Benzol-1,2,3-tricarbonsäure, Benzol-1,2,4-tricarbonsäure (Trimellitsäure), Benzol-1,3,5-tricarbonsäure (Trimesinsäure), Benzol-1,2,3,4-tetracarbonsäure. Weiterhin können Säuren eingesetzt werden, die aus den genannten Säuren durch Substitution eines oder mehrerer am aromatischen Kern gebundenen Wasserstoffatome durch Alkyl-, Cycloalkyl- oder Alkoxyalkylgruppen entstehen.

Es ist möglich, Alkyl-, Cycloalkyl- sowie Alkoxyalkylester z. B. der oben genannten Säuren zu verwenden, wobei diese Reste unabhängig von einander 1 bis 25, insbesondere 3 bis 15, ganz besonders 8 bis 13 C-Atome, insbesondere 9 C-Atome, umfassen. Diese Reste können linear oder verzweigt sein. Hat ein Einsatzprodukt mehr als eine Estergruppe, dann können diese Reste gleich oder verschieden sein.

Im erfindungsgemäßen Verfahren können als Ester einer aromatischen Polycarbonsäure beispielsweise folgende Verbindungen eingesetzt werden: Terephthalsäuremonomethylester, Terephthalsäuredimethylester, Terephthalsäurediethylester, Terephthalsäuredi-n-propylester, Terephthalsäuredibutylester, Terephthalsäurediisobutylester, Terephthalsäuredi-tert.-butylester, Terephthalsäuremonoglykolester, Terephthalsäurediglykolester, Terephthalsäure-n-octylester, Terephthalsäurediisooctylester, Terephthalsäuredi-2-ethylhexylester, Terephthalsäuredi-n-nonylester, Terephthalsäurediisononylester, Terephthalsäuredi-n-decylester, Terephthalsäuredi-n-undecylester, Terephthalsäurediisodecylester, Terephthalsäurediisododecylester, Terephthalsäure-ditridecylester, Terephthalsäuredi-n-octadecylester, Terephthalsäurediisooctadecylester, Terephthalsäuredi-n-eicosylester, Terephthalsäuremonocyclohexylester; Phthalsäuremonomethylester, Phthalsäuredimethylester, Phthalsäuredi-n-propylester, Phthalsäuredi-n-butylester, Phthalsäurediisobutylester, Phthalsäuredi-tert.-butylester, Phthalsäuremonoglykolester, Phthalsäurediglykolester, Phthalsäuredi-n-octylester, Phthalsäurediisooctylester, Phthalsäuredi-2-ethylhexylester, Phthalsäuredi-n-nonylester, Phthalsäurediisononylester, Phthalsäuredi-n-decylester, Phthalsäuredi-2-propylheptylester, Phthalsäurediisodecylester, Phthalsäuredi-n-undecylester, Phthalsäurediisoundecylester, Phthalsäureditridecylester, Phthalsäuredi-n-octadecylester, Phthalsäurediisooctadecylester, Phthalsäuredi-n-eicosylester, Phthalsäuremonocyclohexylester; Phthalsäuredicyclohexylester, Isophthalsäuremonomethylester, Isophthalsäuredimethylester, Isophthalsäuredimethylester, Isophthalsäurediethylester, Isophthalsäuredi-n-propylester, Isophthalsäuredi-n-butylester, Isophthalsäurediisobutylester, Isophthalsäuredi-tert.-butylester, Isophthalsäuremonoglykolester. Isophthalsäurediglykolester, Isophthalsäuredi-n-octylester, Isophthalsäurediisooctylester, Isophthalsäuredi-2-ethylhexylester, Isophthalsäuredi-n-nonylester, Isophthalsäurediisononylester, Isophthalsäuredi-n-decylester, Isophthalsäurediisodecylester, Isophthalsäuredi-n-undecylester, Isophthalsäurediisododecylester, Isophthalsäuredi-n-dodecylester, Isophthalsäureditridecylester, Isophthalsäuredi-n-octadecylester, Isophthalsäurediisooctadecylester, Isophthalsäuredi-n-eicosylester, Isophthalsäuremonocyclohexylester.

Das erfindungsgemäße Verfahren ist prinzipiell auch auf Benzoesäure und deren Ester anwendbar. Hierunter werden Benzoaten von Diolen, wie beispielsweise Glycoldibenzoat, Diethylenglycolbenzoat, Triethylenglycoldibenzoat oder Propylenglycoldibenzoat, auch Benzoesäurealkylester verstanden. Die Alkoholkomponente der Benzoesäurealkylester kann aus 1 bis 25, bevorzugt 8 bis 13 Kohlenstoffatom(en) bestehen. Die Alkohole können linear oder verzweigt sein.

Es können auch Gemische aus zwei oder mehreren Polycarbonsäureestern eingesetzt werden. Solche Gemische können beispielsweise auf folgenden Wegen erhalten werden:
a) eine Polycarbonsäure wird mit einem Alkohol derart partiell verestert, dass Voll- und Partialester nebeneinander vorliegen.
b) Ein Gemisch von mindestens zwei Polycarbonsäuren wird mit einem Alkohol verestert, wobei ein Gemisch von mindestens zwei Vollestern entsteht.
c) Eine Polycarbonsäure wird mit einem Alkoholgemisch versetzt, wobei ein entsprechendes Gemisch der Vollester entstehen kann.
d) Eine Polycarbonsäure wird mit einem Alkoholgemisch partiell verestert.
e) Ein Gemisch von mindestens zwei Carbonsäuren wird mit einem Alkoholgemisch partiell verestert.
f) Ein Gemisch aus mindestens zwei Polycarbonsäuren wird mit einem Alkoholgemisch partiell verestert.

Bei den Umsetzungen a) bis f) können an Stelle der Polycarbonsäuren auch die entsprechenden Anhydride eingesetzt werden.
Großtechnisch werden aromatische Ester, insbesondere die Vollester auf Weg c) häufig aus Alkoholgemischen hergestellt.
Entsprechende Alkoholgemische sind beispielsweise:
C₅-Alkoholgemische, hergestellt aus linearen Butenen durch Hydroformylierung und anschließender Hydrierung;
C₅-Alkoholgemische, hergestellt aus Butengemischen, die lineare Butene und Isobuten enthalten, durch Hydroformylierung und anschließende Hydrierung; C₆-Alkoholgemische, hergestellt aus einem Penten oder aus einem Gemisch von zwei oder mehreren Pentenen, durch Hydroformylierung und anschließende Hydrierung;
C₇-Alkoholgemische, hergestellt aus Triethylen oder Dipropen oder einem Hexenisomer oder einem sonstigen Gemisch von Hexenisomeren, durch Hydroformylierung und anschließende Hydrierung;
C₈-Alkoholgemische, wie 2-Ethylhexanol (2 Isomere), hergestellt durch Aldolkondensation von n-Butyraldehyd und anschließende Hydrierung;
C₉-Alkoholgemische, hergestellt aus C₄-Olefinen durch Dimerisierung, Hydroformylierung und Hydrierung. Dabei kann zur Herstellung der C₉-Alkohole von Isobuten oder von einem Gemisch linearer Butene oder von Gemischen mit linearen Butenen und Isobuten ausgegangen werden. Die C₄-Olefine können mit Hilfe unterschiedlicher Katalysatoren dimerisiert werden, wie beispielsweise Protonensäuren, Zeolithe, metallorganische Nickelverbindungen oder feste nickelhaltige Kontakte. Die Hydroformylierung der C₈-Olefingemische kann mit Hilfe von Rhodium- oder Kobaltkatalysatoren erfolgen. Es gibt daher ein Vielzahl von technischen C₉-Alkoholgemischen.
C₁₀-Alkoliolgemische, hergestellt aus Tripropylen durch Hydroformylierung und anschließende Hydrierung; 2-Propylheptanol (2 Isomere), hergestellt durch Aldolkondensation von Valeraldehyd und anschließende Hydrierung;
C₁₀-Alkoholgemische, hergestellt aus einem Gemisch von mindestens zwei C₅-Aldehyden durch Aldolkondensation und anschließende Hydrierung;
C₁₃-Alkolgemische, hergestellt aus Hexaethylen, Tetrapropylen oder Tributen durch Hydroformylierung und anschließende Hydrierung.

Weitere Alkoholgemische können durch Hydroformylierung und anschließende Hydrierung aus Olefinen bzw. Olefingemischen gewonnen werden, die beispielsweise bei Fischer-Tropsch-Synthesen, bei Dehydrierungen von Kohlenwasserstoffen, Metathesereaktionen, beim Polygasverfahren oder anderen technischen Prozessen anfallen.
Darüber hinaus können auch Olefingemische mit Olefinen unterschiedlicher C-Zahlen für die Herstellung von Alkoholgemischen eingesetzt werden.

Im erfindungsgemäßen Verfahren können alle Estergemische, hergestellt aus aromatischen Polycarbonsäuren und den oben genannten Alkoholgemischen, eingesetzt werden. Erfindungsgemäß werden bevorzugt Ester, hergestellt aus Phthalsäure oder Phthalsäureanhydrid und einem Gemisch isomerer Alkohole mit 6 bis 13 C-Atomen, eingesetzt.

### Beispiele für technische Phthalate, die im erfindungsgemäßen Verfahren eingesetzt werden können, sind folgende Produkte mit den Handelsnamen:

Vestinol C (Di-n-butylphthalat) (CAS Nr.84-74-2); Vestinol IB (Di-i-butylphthalat) (CAS Nr. 84-69-5); Jayflex DINP (CAS Nr.68515-48-0 ); Jayflex DIDP (CAS Nr.68515-49-1); Palatinol 9P (68515-45-7), Vestinol 9 (CAS Nr. 28553-12-0); TOTM (CAS Nr. 3319-31-1); Linplast 68-TM , Palatinol N (CAS Nr. 28553-12-0); Jayflex DHP (CAS Nr. 68515-50-4); Jayflex DIOP (CAS Nr. 27554-26-3); Jayflex UDP (CAS Nr. 68515-47-9); Jayflex DIUP (CAS Nr. 85507-79-5); Jayflex DTDP (CAS Nr.68515-47-9); Jayflex L9P (CAS Nr. 68515-45-7); Jayflex L911P (CAS Nr. 68515-43-5); Jayflex L11P (CAS Nr. 3648-20-2); Witamol 110 (CAS Nr. 68515-51-5); Witamol 118 ( Di-n-C8-C10-alkylphthalat) (CAS Nr.71662-46-9); Unimoll BB (CAS Nr. 85-68-7); Linplast 1012 BP (CAS Nr. 90193-92-3); Linplast 13XP (CAS Nr.27253-26-5); Linplast 610P (CAS Nr. 68515-51-5); Linplast 68 FP (CAS Nr. 68648-93-1); Linplast 812 HP (CAS Nr. 70693-30-0); Palatinol AH (CAS Nr. 117-81-7); Palatinol 711 (CAS Nr. 68515-42-4); Palatinol 911 (CAS Nr. 68515-43-5); Palatinol 11 (CAS Nr. 3648-20-2); Palatinol Z (CAS Nr.26761-40-0); Palatinol DIPP (CAS Nr. 84777-06-0); Jayflex 77 ( CAS Nr. 71888-89-6); Palatinol 10 P (CAS Nr. 53306-54-0); Vestinol AH (CAS Nr. 117-81-7).

Es sei darauf hingewiesen, dass bei der Kernhydrierung aromatischer Polycarbonsäuren bzw. ihrer Ester aus jedem eingesetzten Isomer mindestens zwei stereoisomere Hydrierprodukte entstehen können. Die Mengenverhältnisse der dabei entstandenen Stereoisomeren zueinander hängen vom verwendeten Katalysator und von den Hydrierbedingungen ab.

Alle Hydrierprodukte mit beliebigen Verhältnis(sen) der Stereoisomeren zueinander können ohne Auftrennung verwendet werden.

Die erfindungsgemäß hergestellten alicyclischen Polycarbonsäureester können als Weichmacher in Kunstsoffen verwendeten. Bevorzugte Kunststoffe sind PVC, Homo- und Copolymere auf Basis von Ethylen, Propylen, Butadien, Vinylacetat, Glycidylacrylat, Glycidylmethacrylat, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, Homo- oder Copolymere von cyclischen Olefinen.
Als Vertreter der obigen Gruppen seien beispielsweise folgende Kunststoffe genannt: Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest und Isononylrest, Polymethacrylat, Polymethylmethacrylat, Methylacrylat-Butylacrylat-Copolymere, Methylmethacrylat-Butylmethacrylat-Copolymere, EthylenVinylacetat-Copolymere, chloriertes Polyethylen, Nitrilkautschuk, Acrylnitril-Butadien-StyrolCopolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere und/oder Nitrocellulose.

Darüber hinaus können die erfindungsgemäß hergestellten alicyclischen Polycarbonsäureester zur Modifizierung von Kunststoffmischungen, beispielsweise der Mischung eines Polyolefins mit einem Polyamid, eingesetzt werden.
Gemische aus Kunststoffen und den erfindungsgemäß hergestellten alicyclischen Polycarbonsäureestern sind ebenfalls Gegenstand der vorliegenden Erfindung. Geeignete Kunststoffe sind die bereits genannten Verbindungen. Solche Gemische enthalten bevorzugt mindestens 5 Gew.-%, besonders bevorzugt 20-80 Gew.-%, ganz besonders bevorzugt 30-70 Gew.-% der alicyclischen Polycarbonsäureester.
Gemische aus Kunststoffen, insbesondere PVC, die einen oder mehrere der erfindungsgemäß hergestellten alicyclischen Polycarbonsäureester enthalten, können beispielsweise in folgenden Produkten enthalten sein, bzw. zu deren Herstellung verwendet werden:
Gehäuse für Elektrogeräte, wie beispielsweise Küchengeräte, Computergehäuse, Gehäuse und Bauteile von Phono- und Fernsehgeräte, Rohrleitungen, Apparate, Kabel, Drahtummantelungen, Isolierbändern, Fensterprofilen, im Innenausbau, im Fahrzeug- und Möbelbau, Plastisole, in Bodenbelägen, medizinische Artikel, Lebensmittelverpackungen, Dichtungen, Folien, Verbundfolien, Schallplatten, Kunstleder, Spielzeug, Verpackungsbehälter, Klebebandfolien, Bekleidung, Beschichtungen, als Fasern für Gewebe.

Neben den obengenannten Anwendungen können die erfindungsgemäß hergestellten alicyclischen Polycarbonsäureester als Schmierölkomponente, als Bestandteil von Kühlflüssigkeiten und Metallbearbeitungsflüssigkeiten verwendet werden. Ebenso können sie als Komponente in Farben, Lacken, Tinten und Klebstoffen eingesetzt werden.

Die folgenden Beispiele sollen die Erfindung erläutern, ohne deren Anwendungsbreite, die sich aus der Beschreibung und den Patentansprüchen ergibt, einzuschränken.

### Beispiel 1

### Herstellung eines Titandioxid-Trägers für einen erfindungsgemäßen Katalysator

Eine kommerziell erhältliche Metatitansäure (H₂TiO₃), eine wässrige Suspension, die formal 30 Massen-% Titandioxid und 11 Massen-% Schwefelsäure enthält, wurde mit Hilfe einer Filterpresse filtriert. Je 1 kg eingesetzte Suspension wurde der Filterkuchen mit 5000 g Wasser gewaschen. Der feuchte Filterkuchen hatte formal einen Gehalt von 41,5 Massen-% Titandioxid und von 5,3 Massen-% Schwefelsäure.
Zu einer Suspension von 3000 g Filterkuchen und 3500 g Wasser in einem 10 1-Rührkessel wurden bei Raumtemperatur 85 g Bariumnitrat gegeben. Durch die Zugabe des Bariumnitrats wurde ein Teil der Schwefelsäure zu Bariumsulfat umgesetzt. Nach einstündigem Rühren bei Raumtemperatur wurde die Suspension filtriert. Der erhaltene Filterkuchen wurde fünf Stunden lang bei 110 °C getrocknet und anschließend 3 Stunden lang bei 520°C kalziniert. Dabei wird der Gehalt an "freiem Sulfat" in Form von Schwefeltrioxid weiter reduziert.

Der erhaltene Feststoff enthielt formal 91,5 Massen-% Titandioxid, 5,5 Massen-% Bariumsulfat und 3,1 Massen-% freies Sulfat. Dieser Feststoff wurde zu zwei Pulvertypen aufgemahlt und die gewünschte Korngrößenfraktion ausgesiebt. Das eine Pulver bestand aus Teilchen mit Korngrößen von 1 bis 30 µm, das andere Pulver aus Teilchen mit Korngrößen von 20 bis 500 µm. Die beiden verschiedenen Pulvertypen wurden im Verhältnis 1/1 abgemischt. Zu je 1 kg Pulvergemisch wurden 13g Polyethylenoxid, Typ Polyoxid WSR 301, (Lieferfirma Union Carbide Corporation), 13 g Methylcellulose Culminal MPHC 50 (Lieferfirma Aqualon-Hercules GmbH), 70 g Glasfasern, Typ 8031, (Lieferfirma Bayer AG), 70 g Ethylenglykol und 200 g Wasser gegeben. Das entstandene Gemenge wurde in einem Kneter homogenisiert und anschließend mit Hilfe eines Extruders in Strangextrudaten in Form eines Zylinders mit einem Kreisdurchmesser von 1,5 mm und einer Länge von 4 bis 6 mm geformt. Die Strangextrudate wurden drei Stunden lang bei 80 °C getrocknet und anschließend fünf Stunden lang bei 450 °C kalziniert.

Die physikalischen Kenngrößen des nach Beispiel 1 hergestellten Trägers A für den erfindungsgemäßen Katalysator wurden in Tabelle 1 zusammengestellt. Als Vergleich wurden die Kenngrößen eines technisch häufig eingesetzten Trägers B, aus dem kein erfindungsgemäßer Katalysator hergestellt werden kann, aufgelistet.

**Tabelle 1: Eigenschaften der verwendeten Träger**

| **Material** | **BET-Oberfläche in g/m² nach DIN 66131 (N₂-Adsorption** | **mittlerer Porendurchmesser in nm nach DIN 66133 (Hg-Porosimetrie)** | **Gesamtporenvolumen in ml/g** | **Anteil des Porenvolumens der Makroporen in %** | **Anteil des Porenvolumens der Summe aus Meso- und Mikroporen in** % | **Hersteller oder Typenbezeichnung** |
|---|---|---|---|---|---|---|
| A: TiO₂ | 75 | 14,8 | 0,33 | < 2 | > 98 | H 9063 |
| B: α-Al₂O₃ nicht erfinderisch | 7 | 206,5 | 0,64 | > 97 | < 3 | Axence SP 512 |

| | | | | | | |
|---|---|---|---|---|---|---|
| Das Gesamtporenvolumen wurde aus der Summe der Porenvolumina der Poren mit einem Porendurchmesser > 7,6 nm (bestimmt mit der Hg Porosimetrie) und Poren mit einem Porendurchmesser < 7,6 nm (bestimmt mit der N₂-Adsorptionsmethode) ermittelt. | | | | | | |

### Beispiel 2 und 3

### Herstellung der Hydrierkatalysatoren A und B

Für die Herstellung von Hydrierkatalysatoren auf der Basis der in Tabelle 1 aufgeführten Träger, wurden die Träger zuerst bei 80 °C getrocknet. Nach der Trocknung wurden die Träger mit einer wässrigen Ruthenium (III)-Nitrat-Lösung, die eine Konzentration von 0,8 Gew.-% Ruthenium enthielt, getränkt oder sprühgetrocknet.

Für die Tränkung des Trägers wurde die salpetersaure Ru-Lösung mit Wasser auf ein dem Porenvolumen des Trägers entsprechendes Volumen verdünnt.

Das Aufbringen der Ru-Lösung auf das Trägermaterial erfolgte durch Auftropfen oder vorzugsweise durch gleichmäßiges Versprühen unter Umwälzung des Trägers. Nach der Trocknung bei 120 °C unter Stickstoff wurde der mit Ruthenium-Salz belegte Träger im Wasserstoff/Stickstoff-Gemisch (Verhältnis 1 : 9) bei 200 °C über 6 Stunden lang aktiviert (reduziert).

Anmerkung: Die so hergestellten Katalysatoren wurden im folgenden Text mit dem gleichen Großbuchstaben wie der zugrundeliegende Träger bezeichnet, wobei in einer nachgestellten Klammer das Aktivmetall und dessen Gehalt angegeben wurde.

### Beispiele 3 - 6

### Die Hydrierversuche wurden nach folgender allgemeinen Vorschrift durchgeführt:

90,7 g des Katalysators wurden in einem Katalysatorkörbchen vorgelegt, in einem 1000 ml-Druckreaktor sorgfältig nach obiger Vorschrift im Wasserstoffstrom reduziert und dann mit 590 g flüssigem Diisononylphthalat (Vestinol 9, OXENO Olefinchemie GmbH) versetzt. Die Hydrierung des DINP erfolgte mit reinem Wasserstoff. Nach Hydrierung des Einsatzstoffes wurde der Reaktor entspannt und das Reaktionsgemisch mittels Gaschromatographie auf seinen Gehalt am Zielprodukt Cyclohexan-1,2-dicarbonsäurediisononylester (DINCH) analysiert. Der Umsatz an DINP lag danach stets über 99,9 %.

Die Versuchsbedingungen der Hydrierbeispiele und deren Ergebnisse wurde in Tabelle 2 zusammengefasst:

**Tabelle 2: DINP-Hydrierung / Hydrierbeispiele**

| **Hydrierbeispiele** | **Katalysator** | **Edukt** | **Druck in bar** | **Temperatur in °C** | **Reaktionszeit in Stunden** | **Gehalt an DINCH in** % |
|---|---|---|---|---|---|---|
| 3 | A (1% Ru) | DINP | 200 | 80 | 2,5 | 99,4 |
| 4 | A (1% Ru) | DINP | 200 | 100 | 1 | 99,3 |
| 5 | A (1% Ru) | DINP | 50 | 120 | 0,8 | 99,6 |
| 6 Vergleichsbeispiel | B (1% Ru) | DINP | 200 | 80 | 20 | 99,4 |

Es kann somit gezeigt werden, dass der erfindungsgemäße Katalysator vom Typ A eine deutlich höhere Hydrieraktivität zeigt als Katalysator B.

## Patentansprüche

1. Katalysator zur Hydrierung aromatischer Poly- und/oder Monocarbonsäuren oder deren Derivate zu den entsprechenden alicyclischen Verbindungen, der mindestens ein Metall der achten Nebengruppe des Periodensystems der Elemente auf oder in einem Trägermaterial enthält,
**dadurch gekennzeichnet,**
**dass** das Trägermaterial aus Titandioxid besteht, welches zusätzlich Alkalimetalle, Erdalkalimetalle und/oder Schwefel enthalten kann, und einen mittleren Porendurchmesser von 2 bis 50 nm aufweist und dass über 95 % des Gesamtporenvolumens der Trägermaterialien auf Poren mit einem Durchmesser von kleiner 50 nm entfällt.

2. Katalysator nach Anspruch 1,
**dadurch gekennzeichnet,**
**dass** die spezifische Oberfläche des Trägermaterials zwischen 1 und 350 m²/g beträgt.

3. Katalysator nach Anspruch 1 oder 2,
**dadurch gekennzeichnet,**
**dass** das Trägermaterial aus Titandioxid mit einem Gehalt von 1,0 bis 5,5 Massen-% an freiem Sulfat besteht.

4. Katalysator nach Anspruch 1 bis 3,
**dadurch gekennzeichnet,**
**dass** das Trägermaterial aus Titandioxid mit einem Gehalt von 1,0 bis 4,0 Massen-% Barium besteht

5. Katalysator nach einem der Ansprüche 1 bis 4,
**dadurch gekennzeichnet,**
**dass** der Katalysator zusätzlich mindestens ein Metall der ersten Nebengruppe des Periodensystems der Elemente enthält.

6. Katalysator nach einem der Ansprüche 1 bis 5,
**dadurch gekennzeichnet,**
**dass** der Katalysator zusätzlich mindestens ein Metall der siebten Nebengruppe des Periodensystems der Elemente enthält.

7. Verfahren zur katalytischen Hydrierung von aromatischen Poly- und /oder Monocarbonsäureverbindungen zu den entsprechenden alicyclischen Verbindungen mit Wasserstoff-haltigen Gasen an einem Katalysator, der mindestens ein Metall der achten Nebengruppe des Periodensystems der Elemente auf oder in einem Trägermaterial enthält,
**dadurch gekennzeichnet,**
**dass** das Trägermaterial aus Titandioxid besteht, welches zusätzlich Alkalimetalle, Erdalkalimetalle und/oder Schwefel enthalten kann, und einen mittleren Porendurchmesser von 2 bis 50 nm aufweist und dass über 95 % des Gesamtporenvolumens der Trägermaterialien auf Poren mit einem Durchmesser von kleiner 50 nm entfällt.

8. Verfahren nach Anspruch 7,
**dadurch gekennzeichnet,**
**dass** die spezifische Oberfläche des Trägermaterials zwischen 1 und 350 m²/g beträgt.

9. Verfahren nach Anspruch 7 oder 8,
**dadurch gekennzeichnet,**
**dass** das Trägermaterial aus Titandioxid mit einem Gehalt von 1,0 bis 5,5 Massen-% an freiem Sulfat besteht.

10. Verfahren nach Anspruch 7 bis 9,
**dadurch gekennzeichnet,**
**dass** das Trägermaterial aus Titandioxid mit einem Gehalt von 1,0 bis 4,0 Massen-% Barium besteht.

11. Verfahren nach einem der Ansprüche 7 bis 10,
**dadurch gekennzeichnet,**
**dass** der Katalysator zusätzlich mindestens ein Metall der ersten Nebengruppe des Periodensystems der Elemente enthält.

12. Verfahren nach einem der Ansprüche 7 bis 11,
**dadurch gekennzeichnet,**
**dass** der Katalysator zusätzlich mindestens ein Metall der siebten Nebengruppe des Periodensystems der Elemente enthält.

13. Verfahren nach einem der Ansprüche 1 bis 12,
**dadurch gekennzeichnet,**
**dass** als aromatische Verbindung Benzol-, Diphenyl-, Naphthalin-, Diphenyloxid- oder Anthracencarbonsäure, deren Anhydride und/oder die entsprechenden Ester eingesetzt werden.

14. Verfahren nach Anspruch 13,
**dadurch gekennzeichnet,**
**dass** die Alkoholkomponenten der Carbonsäureester Alkoxyalkyl-, Cycloalkyl-, und/oder Alkylgruppen mit 1 bis 25 Kohlenstoffatomen, verzweigt oder unverzweigt, jeweils gleich oder unterschiedlich sind.

## Claims

1. Catalyst for hydrogenating aromatic polycarboxylic acids and/or monocarboxylic acids or the derivatives thereof to the corresponding alicyclic compounds, said catalyst comprising at least one metal of the eighth transition group of the Periodic Table on or in a support material,
**characterized in that**
the support material consists of titanium dioxide which may additionally comprise alkali metals, alkaline earth metals and/or sulfur and has an average pore diameter of from 2 to 50 nm and **in that** over 95% of the total pore volume of the support materials is accounted for by pores having a diameter of less than 50 nm.

2. Catalyst according to Claim 1,
**characterized in that**
the specific surface area of the support material is between 1 and 350 m²/g.

3. Catalyst according to Claim 1 or 2,
**characterized in that**
the support material consists of titanium dioxide having a content of from 1.0 to 5.5% by mass of free sulfate.

4. Catalyst according to Claims 1 to 3,
**characterized in that**
the support material consists of titanium dioxide having a content of from 1.0 to 4.0% by mass of barium.

5. Catalyst according to Claims 1 to 4,
**characterized in that**
the catalyst additionally comprises at least one metal of the first transition group of the Periodic Table.

6. Catalyst according to Claims 1 to 5,
**characterized in that**
the catalyst additionally comprises at least one metal of the seventh transition group of the Periodic Table.

7. Process for catalytically hydrogenating aromatic polycarboxylic acid compounds and/or monocarboxylic acid compounds to the corresponding alicyclic compounds using hydrogen-containing gases over a catalyst which comprises at least one metal of the eighth transition group of the Periodic Table on or in a support material,
**characterized in that**
the support material consists of titanium dioxide which may additionally comprise alkali metals, alkaline earth metals and/or sulfur and has an average pore diameter of from 2 to 50 nm and **in that** over 95% of the total pore volume of the support materials is accounted for by pores having a diameter of less than 50 nm.

8. Process according to Claim 7,
**characterized in that**
the specific surface area of the support material is between 1 and 350 m²/g.

9. Process according to Claim 7 or 8,
**characterized in that**
the support material consists of titanium dioxide having a content of from 1.0 to 5.5% by mass of free sulfate.

10. Process according to Claims 7 to 9,
**characterized in that**
the support material consists of titanium dioxide having a content of from 1.0 to 4.0% by mass of barium.

11. Process according to Claims 7 to 10,
**characterized in that**
the catalyst additionally comprises at least one metal of the first transition group of the Periodic Table.

12. Process according to Claims 7 to 11,
**characterized in that**
the catalyst additionally comprises at least one metal of the seventh transition group of the Periodic Table.

13. Process according to any of Claims 1 to 12,
**characterized in that**
the aromatic compound used is a carboxylic acid of benzene, diphenyl, naphthalene, diphenyl oxide or anthracene, its anhydride and/or the corresponding esters.

14. Process according to Claim 13,
**characterized in that**
the alcohol components of the carboxylic esters are alkoxyalkyl, cycloalkyl and/or alkyl groups having from 1 to 25 carbon atoms, branched or unbranched, each the same or different.

## Revendications

1. Catalyseur pour l'hydrogénation d'acides polycarboxyliques et/ou monocarboxyliques aromatiques ou de leurs dérivés en composés alicycliques correspondants, qui contient au moins un métal du huitième groupe secondaire du système périodique des éléments sur ou dans un matériau support, **caractérisé en ce que** le matériau support est en dioxyde de titane qui peut en outre contenir des métaux alcalins, des métaux alcalino-terreux et/ou du soufre et qui présente un diamètre moyen des pores de 2 à 50 nm et **en ce que** les pores présentant un diamètre inférieur à 50 nm constituent plus de 95% du volume total des pores des matériaux support.

2. Catalyseur selon la revendication 1, **caractérisé en ce que** la surface spécifique du matériau support est située entre 1 et 350 m²/g.

3. Catalyseur selon la revendication 1 ou 2, **caractérisé en ce que** le matériau support est constitué par du dioxyde de titane présentant une teneur de 1,0 à 5,5% en masse de sulfate libre.

4. Catalyseur selon la revendication 1 à 3, **caractérisé en ce que** le matériau support est constitué par du dioxyde de titane présentant une teneur de 1,0 à 4,0% en masse de baryum.

5. Catalyseur selon l'une quelconque des revendications 1 à 4, **caractérisé en ce que** le catalyseur contient en outre au moins un métal du premier groupe secondaire du système périodique des éléments.

6. Catalyseur selon l'une quelconque des revendications 1 à 5, **caractérisé en ce que** le catalyseur contient en outre au moins un métal du septième groupe secondaire du système périodique des éléments.

7. Procédé pour l'hydrogénation catalytique d'acides polycarboxyliques et/ou monocarboxyliques aromatiques en composés alicycliques correspondants avec des gaz contenant de l'hydrogène sur un catalyseur, qui contient au moins un métal du huitième groupe secondaire du système périodique des éléments sur ou dans un matériau support, **caractérisé en ce que** le matériau support est en dioxyde de titane qui peut en outre contenir des métaux alcalins, des métaux alcalino-terreux et/ou du soufre et qui présente un diamètre moyen des pores de 2 à 50 nm et **en ce que** les pores présentant un diamètre inférieur à 50 nm constituent plus de 95% du volume total des pores des matériaux support.

8. Procédé selon la revendication 7, **caractérisé en ce que** la surface spécifique du matériau support est située entre 1 et 350 m²/g.

9. Procédé selon la revendication 7 ou 8, **caractérisé en ce que** le matériau support est constitué par du dioxyde de titane présentant une teneur de 1,0 à 5,5% en masse de sulfate libre.

10. Procédé selon la revendication 7 à 9, **caractérisé en ce que** le matériau support est constitué par du dioxyde de titane présentant une teneur de 1,0 à 4,0% en masse de baryum.

11. Procédé selon l'une quelconque des revendications 7 à 10, **caractérisé en ce que** le catalyseur contient en outre au moins un métal du premier groupe secondaire du système périodique des éléments.

12. Procédé selon l'une quelconque des revendications 7 à 11, **caractérisé en ce que** le catalyseur contient en outre au moins un métal du septième groupe secondaire du système périodique des éléments.

13. Procédé selon l'une quelconque des revendications 1 à 12, **caractérisé en ce qu'**on utilise comme composé aromatique de l'acide de benzène, de diphényle, de naphtalène, de diphényloxyde ou d'anthracène, leurs anhydrides et/ou les esters correspondants.

14. Procédé selon la revendication 13, **caractérisé en ce que** les composants alcool des esters d'acides carboxyliques sont des groupes alcoxyalkyle, cycloalkyle et/ou alkyle ramifiés ou non ramifiés, comprenant 1 à 25 atomes de carbone, à chaque fois identiques ou différents.
